(19) Europäisches Patentamt / European Patent Office / Office européen des brevets

(11) **EP 1 557 460 B1**

(12) **EUROPEAN PATENT SPECIFICATION**

(45) Date of publication and mention of the grant of the patent:
**09.12.2015 Bulletin 2015/50**

(51) Int Cl.:
**C12P 7/62** (2006.01)

(21) Application number: **03758720.1**

(22) Date of filing: **10.10.2003**

(86) International application number:
**PCT/JP2003/013022**

(87) International publication number:
**WO 2004/033670 (22.04.2004 Gazette 2004/17)**

(54) **CULTURE METHOD OF CONTROLLING THE COMPOSITION OF COPOLYMER POLYESTER**

KULTIVIERUNGSVERFAHREN ZUR STEUERUNG DER ZUSAMMENSETZUNG EINES COPOLYMER-POLYESTERS

METHODE DE CULTURE PERMETTANT DE REGULER LA COMPOSITION D'UN POLYESTER COPOLYMERE

(84) Designated Contracting States:
**DE FR GB IT**

(30) Priority: **10.10.2002 JP 2002297601**

(43) Date of publication of application:
**27.07.2005 Bulletin 2005/30**

(73) Proprietor: **KANEKA CORPORATION Osaka (JP)**

(72) Inventors:
• **NAKASHIMA, Toshimitsu**
  **Takasago-shi,**
  **Hyogo 676-0035 (JP)**
• **ODAWARA, Osamu**
  **Takasago-shi,**
  **Hyogo 676-0008 (JP)**
• **YOKOMIZO, Satoru**
  **Kobe-shi, Hyogo 653-0854 (JP)**

(74) Representative: **Hoffmann Eitle**
  **Patent- und Rechtsanwälte PartmbB**
  **Arabellastraße 30**
  **81925 München (DE)**

(56) References cited:
EP-A2- 0 824 148    JP-A- 1 027 483
JP-A- 7 155 192     JP-A- 8 289 797
JP-A- 2001 340 078  US-A- 5 292 860
US-A- 5 871 980

• FUKUI T ET AL: "Efficient production of polyhydroxyalkanoates from plant oils by alcaligenes eutrophus and its recombinant strain" APPLIED MICROBIOLOGY AND BIOTECHNOLOGY, SPRINGER VERLAG, BERLIN, DE, vol. 49, no. 3, 1 March 1998 (1998-03-01), pages 333-336, XP002979648 ISSN: 0175-7598
• RAMSAY B A ET AL: "EFFECT OF NITROGEN LIMITATION ON LONG-SIDE-CHAIN POLY-BETA HYDROXYALKANOATE SYNTHESIS BY PSEUDOMONAS-RESINOVORANS" APPLIED AND ENVIRONMENTAL MICROBIOLOGY, vol. 58, no. 2, 1992, pages 744-746, XP002574057 ISSN: 0099-2240
• SUDESH K ET AL: "Synthesis, structure and properties of polyhydroxyalkanoates: biological polyesters" PROGRESS IN POLYMER SCIENCE, PERGAMON PRESS, OXFORD, GB, vol. 25, no. 10, 1 December 2000 (2000-12-01), pages 1503-1555, XP003000229 ISSN: 0079-6700
• WITHOLT B ET AL: "PERSPECTIVES OF MEDIUM CHAIN LENGTH POLY (HYDROXYALKANOATES), A VERSATILE SET OF BACTERIAL BIOPLASTICS" CURRENT OPINION IN BIOTECHNOLOGY, LONDON, GB, vol. 10, 1 January 1999 (1999-01-01), pages 279-285, XP000925867 ISSN: 0958-1669
• SEUNG HWAN LEE ET AL.: 'Production of poly (3-hydroxybutyrate-co-3-hydroxyhexanoat e) by high-cell-density cultivation of aeromonas hydrophilia' BIOTECHNOL. BIOENG. vol. 67, no. 2, 2000, pages 240 - 244, XP002976378

- **CHEN G.Q. ET AL.: 'Industrial scale production of poly(3-hydroxybutyrate-co-3-hydroxyhexanoat e)' APPL. MICROBIOL. BIOTECHNOL. vol. 57, no. 1/2, 2001, pages 50 - 55, XP002976379**

**Description**

TECHNICAL FIELD

The present invention relates to a culture method for controlling a composition of a copolyester produced by a microorganism.

BACKGROUND ART

Up to now, a large number of microorganisms have been known to store polyester as an energy source substance within cells. A typical example of the polyester is poly-3-hydroxybutyric acid (hereinafter referred to briefly as "P (3HB) ") . P (3HB) is a thermoplastic polymer and is biodegradable in the natural environment and, thus, has recently attracted attention as an ecofriendly green plastic. However, since P(3HB) is high in crystallinity, it is hard and fragile by nature, so that the range of practical application thereof is limited. Therefore, studies have been undertaken to modify the P (3HB) for improving these properties.

In the course of the study, a production method of a copolymer P(3HB-co-3HV) derived from 3-hydroxybutyric acid (hereinafter, referred to briefly as "3HB") and 3-hydroxyvaleric acid (hereinafter, referred to briefly as "3HV") have been developed (Japanese Kokai Publication Sho-57-150393, Japanese Kokai Publication Sho-59-220192 and Japanese Kohyo Publication Hei-11-500008). This P(3HB-co-3HV) is rich in flexibility as compared with P(3HB), hence it was considered to have a wide application range. Methods for producing the copolymer described in these patent documents comprise growing cells in the first stage and culturing a microorganism with restricting nitrogen or phosphorus in the latter stage to produce the copolymer similarly to the conventional methods for producing P(3HB). Moreover, as for P (3HB-co-3HV), since the flexibility changes as a content of 3HV increases, researches for controlling the composition ratio of 3HV have also been made. For example, propionic acid is used in Japanese Kokai Publication Sho-57-150393.and Japanese Kokai Publication Sho-63-269989, and propan-1-ol is used in Japanese Kokoku Publication Hei-7-79705, and by changing an addition amount thereof to a medium, the 3HV content is controlled to produce P (3HB-co-3HV) having the 3HV content of 10 to 90 mol%. Actually, however, P (3HB-co-3HV) shows only slight changes in the characteristics even when the 3HV content is increased. In particular; the flexibility is not improved to such an extent required for its use in films and the like. Thus, it has been used only in the field of rigid shaped articles such as shampoo bottles and disposable razor grips.

Under such circumstances, for making up the above-mentioned drawbacks of the copolymer derived from 3HB and 3HV, copolyesters containing, as a component, ahydroxyalkanoic acid other than 3HB and 3HV such as 3-hydroxypropionic acid (hereinafter referred to briefly as "3HP"), 3-hydroxyhexanoic acid (hereinafter referred to briefly as "3HH"), 3-hydroxyoctanoic acid (hereinafter referred to briefly as "3HO"), 3-hydroxynonanoic acid (hereinafter referred to briefly as "3HN"), 3-hydroxydecanoic acid (hereinafter referred to briefly as "3HD") or 3-hydroxydodecanoic acid (hereinafter referred to briefly as "3HDD") are intensively studied (Poirier, Y., Nawrath C., Somerville C, BIO/TECHNOLOGY, 13, 142-150, 1995). Among them, noteworthy studies are those on a copolyester comprising 3HB and 3HH unit, particularly a copolymer P(3HB-co-3HH) derived only from 3HB and 3HH, and on a production method thereof (Japanese Kokai Publication Hei-05-93049 and Japanese Kokai Publication Hei-07-265065). The production methods of P(3HB-co-3HH) described in these patent documents comprise a fermentation production from fatty acids such as oleic acid or oils and fats such as olive oil by using <u>Aeromonas caviae</u> isolated from soil. A study regarding characteristics of P (3HB-co-3HH) has also been conducted (Y. Doi, S. Kitamura, H. Abe, Macromolecules 28, 4822-4823, 1995). This document reports a fermentation production of P (3HB-co-3HH) with a 3HH content of 11 to 19 mol% by culturing <u>A</u>. <u>caviae</u> with a fatty acid of not less than 12 carbon atoms as the only carbon source. The result shows that, as the 3HH content increases, P(3HB-co-3HH) exhibits a gradual increase of flexibility from the hard and brittle characteristics of P(3HB) and finally shows more flexibility than P(3HB-co-3HV).

Additionally, it was reported that a polyhydroxyalkanoic acid (PHA) synthase gene from <u>A. caviae</u> was cloned and introduced into <u>R. eutropha</u> having a high accumulating ability of polyhydroxybutyric acid(PHB) of not less than 90% to generate a recombinant strain, which was then used to produce P (3HB-co-3HH) using fatty acids as a carbon source (T. Fukui, Y. Doi, J. Bacteriol., vol. 179, No. 15, 4821-4830, 1997 and Japanese Kokai Publication Hei-10-108682). In these documents, it is reported that P (3HB-co-3HH) having the 3HH content of 10 to 20 mol% may be produced by using sodium octanoate as a carbon source. The above recombinant strain was also used to produce P (3HB-co-3HH) employing plant oils (T. Fukui, Y. Doi, Appl. Microbiol. Biotechnol., vol. 49, No. 3, 333-336, 1998). In that study, copolyesters with a similar 3HH content of 4-5 mol% were reported, irrespective of the plant oil used. In contrast, a method has been recently disclosed which comprises using multiple carbon sources in producing a polyester using the above recombinant strain, and it was revealed that a carbon number of an oil or fat or a fatty acid used as a carbon source had an influence on the 3HH-content of P(3HB-co-3HH) (Japanese Kokai Publication 2001-340078). Additionally, a study using Aeromonas hydrophila reported that P (3HB-co-3HH) having a 3HH content of 17 mol% may be produced using lauric acid as carbon source (S.H. Lee, D.H. Oh, W.S. Ahn, Biotechnol. Bioeng., vol. 67, No. 3, 240-244, 2000).

If the composition ratio of monomeric units in copolyesters such as P(3HB-co-3HH), particularly the 3HH content may be controlled in an optional wide range in the future, both hard copolymers and soft copolymers may be produced by fermentation, and such copolymers will find a wide range of applications, from chassis for TV-set, which is required to be hard, to a thread or a film, which are required to be flexible.

As described above, with respect to copolyesters such as P (3HB-co-3HH), it is most important to establish a technology capable of arbitrary controlling the composition ratio of the copolyester in order to achieve practical or commercial application as well as to satisfy demands of consumers. Another barrier for the practical application is high production cost. With regard to that, a specific expensive fatty acid is required to be added to a culture medium for copolymerizing monomeric units other than 3HB or for increasing the content thereof in the conventional methods of producing a copolyester (Japanese Kohyo Publication Hei-11-500008 and Japanese Kokai Publication 2001-340078). Moreover, with respect to P(3HB-co-3HH), the yield of cells is low in any methods disclosed hitherto, and not only expensive carbon sources are required but also the productivity tends to be more deteriorated when trying to enhance the 3HH content. Thus, none of the conventional methods can be adopted as a production method for practical application of the polymer. As described above, it is essential to control the composition ratio of the monomeric unit in a copolyester in order to find a wide range of applications for the copolyester. Whereupon, it has been long awaited to develop a technology which may realize a high productivity of cells and polymer content in low cost as well, and which enables to arbitrary control the composition of the copolyester.

SUMMARY OF THE INVENTION

In view of the above-mentioned state of the art, the present invention provides a production method capable of controlling a composition of a biodegradable 3HH-containing copolyester as well as achieving a high productivity in low cost.

The present inventors have made various investigations, and particularly have studied on various fermentation raw materials in view of prices, supply stability, quality stability, yield of cells or polymers and the like. As a result, they have succeeded in culturing a microorganism accumulating a 3HH-containing copolyester with an inexpensive oil or fat as a carbon source while maintaining the high productivity and preferably controlling the monomer composition within an optional range without particularly using an expensive fatty acid and the like, wherein the microorganism is selected from the group consisting of microorganisms belonging to the genus Ralstonia, the genus Pseudomonas, the genus Aeromonas, the genus Alcaligenes and the genus Escherichia.

Thus, the point of the present invention relates to a culture method which comprises controlling a specific substrate feed rate of an oil or fat to be used as a carbon source at a constant value throughout the whole culture period according to claim 1, or dividing the culture period into two phases consisting of a cell growth phase and a polyester accumulation phase and controlling the specific substrate feed rate at a constant value during the respective phases according to claim 2. The present invention further relates to a culture method comprising controlling the composition of the produced 3HH-containing copolyester by selecting the species and/or the control value of the specific substrate feed rate.

DETAILED DESCRIPTION OF THE INVENTION

Hereinafter, the present invention is described in detail.

The culture method for controlling the composition of the 3HH-containing copolyester according to the present invention comprises controlling the specific substrate feed rate of an oil or fat to be used as a carbon source at a constant value throughout the whole culture period according to claim 1, or applying the different rate between the cell growth phase and the polyester accumulation phase and controlling the rate at a constant value during the respective phases according to claim 2. Thus, the said culture method is applied to a production of a biodegradable 3HH-containing copolymer using a microorganism selected from the group consisting of microorganisms belonging to the genus Ralstonia, the genus Pseudomonas, the genus Aeromonas, the genus Alcaligenes and the genus Escherichia.

Although there is no particular limitation on a 3HH-containing copolyester capable of being applied to the culture method of the present invention, as long as it is a copolyester that may be obtained by polymerization of at least two monomeric units. As said copolyester, specifically, there may be mentioned a copolyester P (3HB-co-3HH) derived from 3HB and 3HH; a copolymer derived from three components of 3HB, 3HH and 3HV; and another copolymer containing, as components, multiple elements such as 3HB, 3HH, 3HO, 3HD and 3HDD produced by bacteria belonging to the genus Pseudomonas as typical examples (I.K.P. Tan, K.S. Kumar, M. Theanmalar, S.N. Gan, B. Gordon III, Appl. Microbiol. Biotechnol., 47, 207-211, 1997; R.D. Ashby, T.A. Foglia, Appl. Microbiol. Biotechnol., 49, 431-437, 1998). Among them, a P(3HB-co-3HH) is preferable.

In the culture method according to the present invention, the microorganism to be used is selected from the group consisting of microorganisms belonging to the genus Ralstonia, the genus Pseudomonas, the genus Aeromonas, the genus Alcaligenes and the genus Escherichia.

Moreover, in the case that a wild-type of the above microorganisms cannot produce an objective copolymer, or the case that the production amount is low, the above microorganisms may be transformed by introducing a polymerase gene of the objective copolyester and the obtained transformed microorganism may be used. To produce the transformed microorganism, general methods may be used which, for example, utilizes a recombinant vector containing a polyester polymerase gene. As said recombinant vector, a plasmid vector capable of growing autonomously within the cells may be used. Moreover, said polyester polymerase gene may be directly incorporated into the chromosome of the host. As the host, bacteria belonging to the genus Ralstonia, the genus Pseudomonas, the genus Aeromonas, the genus Alcaligenes, or the genus Escherichia are used.

The polyester polymerase gene to be used in the method for producing a copolyester according to the present invention is not particularly restricted. However, preferred is a gene isolated fromAeromonas caviae, and for example, a gene fragment described in Japanese Kokai Publication Hei-10-108682 may be used.

Additionally, the conventional methods may be applied in introducing the recombinant vector to the microorganism. For example, the conjugation method, the calcium method, the electroporation method and the like may be used.

As a preferable example of the microorganism to be used in the present invention, there may be mentioned a Ralstonia eutropha strain PHB-4/pJRDEE32d13, which is obtained by introducing a polyester polymeraze gene derived from Aeromonas caviae into Ralstonia eutropha (T. Fukui., Y. Doi., Appl. Microbiol. Biotechnol., 49, 333-336 (1998)).

This strain is internationally deposited to the National Institute of Advanced Industrial Science and Technology, International Patent Organism Depositary (Central 6, 1-1-1, Higashi, Tsukuba, Ibaraki, JAPAN) on the date of August 7, 1997 with the name of Alcaligenes eutrophus AC 32 in an accession number of FERM BP-6038 under Budapest treaty.

As a fermentation material used in the culture method of the present invention, an inexpensive oil or fat is used as a main carbon source for producing a copolyester from viewpoints of price, supply stability, quality stability, yield of cells or polymers, and the like. The term "main" as used herein means accounting for not less than 50% of the entire carbon source. As the carbon sources other than oils and fats, saccharides such as glucose, fructose, sucrose and lactose may be used, for example.

A medium containing a nitrogen source, inorganic salts, vitamins and other general organic nutrient sources may be used as a nutrient source other than the carbon source. As the nitrogen source, there may be mentioned inorganic nitrogen sources e.g. ammonia, ammonium salts such as ammonium chloride, ammonium sulfate and ammonium phosphate, and organic nitrogen sources such as peptone, meat extract and yeast extract, for instance. As the inorganic salts, there may be mentioned potassium dihydrogenphosphate, dipotassium hydrogenphosphate, magnesium phosphate, magnesium sulfate or sodium chloride and the like, for instance. As the vitamins, there may be mentioned, for example, vitamin B1, vitamin B12, vitamin C, and the like. As the other organic nutrient sources, there may be mentioned amino acids such as glycine, alanine, serine, threonine, proline and the like, for example. However, from a viewpoint of suppressing the production cost, it is preferable to use only a minimum amount of organic nitrogen sources such as peptone, meat extract and yeast extract; amino acids such as glycine, alanine, serine, threonine and proline; vitamins such as vitamin B1, vitamin B12 and vitamin C. Particularly among them, the amount to be used of peptone, yeast extract, meat extract or the like is more preferable to be kept in a minimum amount.

Generally, a production of polyester by a microorganism is preferably accomplished under the condition in which nutrients necessary for cell growth such as nitrogen and phosphorus are restricted to certain levels. Also in the present invention, such nutrient restriction may be carried out and, inter alia, it is more preferable to restrict nitrogen or phosphorus, and it is still more preferable to restrict only phosphorus without restricting nitrogen. Moreover, "restriction of phosphorus" in the present invention does not mean the condition that completely no phosphorus atom is contained in a medium but means the condition in which phosphorus as a nutrient source is contained in a minimum amount necessary for growth. That is, it means the condition with a growth amount of cells being limited by phosphorus, and does not exclude phosphorus contained in a small amount as an inorganic salt in a medium.

As the oils or fats to be used as carbon sources in the present invention, there may be used natural oils which are supplied in a relatively stable manner such as soybean oil, corn oil, cottonseed oil, palm oil, palm kernel oil, coconut oil and peanut oil; respective fractionated oils and fats obtained by fractioning these oils (for example, "palm W olein oil" (low-melting point fraction obtained by fractionating palm oil twice without solvent), "palm kernel olein oil" (low-melting point fraction obtained by fractionating palm kernel oil once without solvent)) or the like; synthesized oils obtained by treating these natural oils and fractions thereof chemically or biochemicallly; and further mixed oils obtained by mixing these oils. Among them, it is preferable to use natural oils or fats and fractionated oils or fats, and it is more preferable to use natural oils or fats and inexpensive fractionated oils or fats from a viewpoint of economy.

Herein, it is possible to control the species and composition ratio of the monomeric units constituting the copolyester by appropriately selecting the oils or fats to be used. For example, in the case a copolyester with a high 3HH content is desired in producing a copolyester comprising 3HH unit, it is preferable to use an oil or fat containing lauric acid in the constituent fatty acids, which is commonly called as "lauric oils". Oils or fats containing lauric acids in the constituent fatty acid include natural oils and fats such as palm kernel oil and coconut oil, fractionated oils and fats such as palm kernel olein oil, and mixed oils containing these lauric oils and fats.

When the case of P(3HB-co-3HH) is explained more specifically, P (3HB-co-3HH) with a relatively high 3HH content of 4 to 20 mol% may be obtained using lauric oils, and P (3HB-co-3HH) with a relatively low 3HH content of 1 to 10 mol% may be obtained using soybean oil, corn oil, cottonseed oil, palm oil, peanut oil or a fractionated oils of these oils. Furthermore, the 3HH content of P(3HB-co-3HH) may be arbitrary controlled by using a mixed oil obtained by mixing two or more species of these oils and by altering the mixing ratio optionally.

In addition, it is particularly preferable that the oils or fats used as carbon sources contain lauric acid in constituent fatty acids and cultured under the condition in which phosphorus is restricted.

In general, when an oil or fat is added in a fermentation medium, as an addition method thereof, there may be considered methods such as a shot of a large amount at once, an addition in portions and a continuous or intermittent feed. However, as a result of the investigation conducted by the present inventors, it was found that when a large amount is shot at once, toxicity for cells may appear by generated fatty acids, or an actual operation may become difficult owing to foaming attributed to the fatty acids generated. Therefore, in the present invention, a method comprising feeding an oil or fat as a carbon source continuously or intermittently using a pump and the like is selected. As a result of the investigation conducted by the present inventors for researching the most appropriate addition method of an oil or fat as a carbon source, they found that not only operational problems such as foaming may be prevented but also the composition of the produced copolyester may be controlled by designing the addition method. In the following, the addition method of an oil or fat carried out in the culture method of the present invention is described specifically.

In the culture method according to the present invention, the oils or fats as carbon sources are fed in a manner that the specific substrate feed rate of the oils or fats is set at an almost constant value throughout the whole culture period or during the respective phases of the cell growth phase and the polyester accumulation phase.

As used herein, the term "specific substrate feed rate" means an amount of an oil or fat supplied per net weight of cells during a unit time, that is, a culture parameter defined as a feed rate of an oil or fat per net weight of cells. The term "net weight of cell" means the weight of the cells (weight of dried cells) calculated by subtracting the weight of polyester contained in the cells from the weight of the whole cells. Thus, the specific substrate feed rate is calculated according to the following formula (1):

```
(Specific substrate feed rate)
= (Feed rate of an oil or fat (g/h)) / (Net weight of cells (g))
= (Supply amount of an oil or fat per hour (g/h))/[(Whole cell
weight (g)) - (Polyester contained in cells (g))]
```

In the present invention, in order to set the specific substrate feed rate and to control the rate at a constant value, it is necessary to predict a change in the net weight of cells. The present inventors have extensively studied on the changes in the net weight of cells, and have succeeded in predicting the growth curve of the net weight of cells, which may be used in practical applications, through preliminary experiments, and finally completed the present invention.

As a result of the investigation conducted by the present inventors, the holdup of an oil or fat exist in a culture broth (volume ratio of the oil or fat accounting for the whole culture broth (medium + cells + oil or fat) is as small as not more than 10% within the feed rate of the oil or fat capable of realizing a stable culture, and there seems no particular change in a droplet diameter of the oil or fat determined by stirring and aeration conditions. Thus, it is presumed that there is no significant difference in a rate of substrate uptake, which is governed by the interface area of a droplet diameter of the oil and fat and, and as a result if the species of the oil or fat and other culture conditions are the same, changes in the net weight of cells may be approximately represented by a single growth curve within the range of the feed rate of the oil or fat capable of stable operation.

Moreover, since the production of a biodegradable polyester is conducted under the condition nitrogen or phosphate being limited as described below, the net weight of cells does not almost change and becomes constant after the depletion of nitrogen or phosphorus. Accordingly, once the species of an oil or fat and culture conditions are determined, the feed rate of the oil or fat may be appropriately changed under that condition within the range that the supply of the oil or fat is not insufficient but is not excess (in the actual operation, the feed rate is adjusted in accordance with the observation of the thickness of the oil or fat layer in the culture supernatant). Data on the changes in the net weight of cells may thus be collected. In this way, the growth curve of the net weight of cells may be obtained. On the basis of the growth curve, an addition amount of the oil or fat required for maintaining the predetermined constant value of the specific substrate feed rate during the culture period or during the period of a particular phase as described above may be calculated based on the above formula (1) . Then, in accordance with the calculation results, the oil or fat may be fed by changing the feed rate continuously (stepwisely) or intermittently.

Furthermore, as an indirect method, there may be mentioned a method comprising measuring an oxygen concentration and a carbon dioxide concentration in an exhaust gas to estimate the net weight of cells by the oxygen consuming rate or the carbon dioxide generation rate to control the specific substrate feed rate on a real-time basis. However, as a result of the investigation conducted by the present inventors, a significant change is acknowledged in the above respiration property between the cell growth phase, in which nitrogen or phosphorus does not become depleted, and the polyester accumulation phase, which is after depletion. Therefore, it is preferable to study the respiration property in the growth phase and the production phase in detail beforehand.

In the present invention, either of the following methods may be used to control the specific substrate feed rate at a certain constant value: a method comprising controlling the specific substrate feed rate at a constant value throughout the whole culture period, and a method comprising dividing the whole culture period into two phases of the cell growth phase and the polyester accumulation phase and controlling the specific substrate feed rate at a constant value separately so as to be the set value during the respective phases. To control the specific substrate feed rate at a constant value throughout the whole culture period or during a particular phase, it is required to change the feed rate of the oil or fat continuously (stepwisely) or intermittently so as to satisfy the set value of the specific substrate feed rate.

For changing the feed rate of the oil or fat continuously (stepwisely), the rate may be controlled by using a computer, for example . Although an automatic control may be possible when the rate is changed intermittently or stepwisely, a manual operation of the feed rate is also possible; thus it is convenient. For intermittent or stepwise alteration, it will be sufficient if the preset specific substrate feed rate is fulfilled as an average over the supply period even if the rate is not always completely fulfilled in a strict sense.

As described above, by the method of the present invention which comprises controlling the specific substrate feed rate at a constant value throughout the whole culture period or during a particular period of the whole culture period, an improvement of the productivity of copolyester and control on the composition ratio of monomer units may be achieved for the first time. The conventional culture methods, such as that total of the predetermined amount of oil or fat should be shotted in an early stage of a culture, that fed-batch culture should be performed with a constant feed rate of oil or fat during the whole culture period, and that an addition amount of oil or fat is altered based on experiences instead of using the specific substrate feed rate, have been inadequate to such improvement of the productivity and the composition control of polyester.

In this specification, the term "throughout the whole culture period" refers to the whole period of the main culture for the production of the polyester from the beginning to end of the culture. The term "controlling the specific substrate feed rate at a constant value throughout the whole culture period" means selecting the same constant value of the specific substrate feed rate and controlling the feed rate of the oil or fat at the value during both of the cell growth phase and polyester accumulation phase. As used herein, the cell growth phase and polyester accumulation phase of the culture indicate the first phase (the cell growth phase), in which there is sufficient amount of nitrogen or phosphate in a medium, the cells grow actively, and the polyester accumulation rate is not so high, and the latter phase (the polyester accumulation phase), in which the concentration of nitrogen or phosphate is reduced in the medium, cell proliferation is limited, and the polyester accumulation rate increases, respectively, when the culture period is roughly divided into two phases. The improvement of the productivity of the copolyester and control of the composition ratio of monomeric units may be achieved more efficiently by setting different values of the specific substrate feed rate for each culture phase and carrying out the culture with controlling the specific substrate feed rate at a constant value set individually for each phase during the culture.

Although the range of the specific substrate feed rate applied in the method of the present invention differs depending on the species of the oil or fat used and on the phase of the culture, it is typically 0.05 to 0.20 (g; oil or fat) $\times$ (g; net weight of dried cells)$^{-1}$ $\times$ (hour) $^{-1}$, preferably 0.06 to 0.15 (g; oil or fat) $\times$ (g; net weight of dried cells)$^{-1}$ $\times$ (hour)$^{-1}$, and more preferable 0. 07 to 0. 12 (g; oil or fat) $\times$ (g; net weight of dried cells)$^{-1}$ $\times$ (hour)$^{-1}$.

In the present invention, it has been first discovered that the 3HH content of P (3HB-co-3HH) increases as the specific substrate feed rate decreases. Thus, if P(3HB-co-3HH) with a high 3HH content is desired, the culture may be carried out with setting a low specific substrate feed rate (for example, 0.06 to 0.08 (g; oil) $\times$ (g; net weight of dried cells)$^{-1}$ $\times$ (hour)$^{-1}$). In addition, if specific substrate feed rate is altered depending on a phase of the culture, the 3HH content may be enhanced more efficiently without decreasing the productivity of the copolyester by setting the specific substrate feed rate to be higher during the cell growth phase (for example, 0.09 to 0.13 (g; oil) $\times$ (g; net weight of dried cells)$^{-1}$ $\times$ (hour)$^{-1}$) and the rate during the polyester accumulation phase to be lower (for example, 0.06 to 0.08 (g; oil) $\times$ (g; net weight of dried cells)$^{-1}$ $\times$ (hour)$^{-1}$).

Furthermore, as described above, it is possible to control the composition of the produced copolyester by appropriately selecting the species of oils and fats. Accordingly, it becomes possible to control the composition of the produced copolyester, for example, the 3HH content of P (3HB-co-3HH) and the like to the desired composition ratio by altering the species of the oil or fat, the control value of the specific substrate feed rate or further selecting a preferable combination of these.

The culture temperature of the microorganism may be any temperature as long as the cells may grow, but preferably at 20 to 40°C, more preferably at 25 to 35°C. The culture period is not particularly restricted and may be 1 to 7 days, preferably 40 to 70 hours.

The above explained control of the specific substrate feed rate, selection of the species of the oil or fat, restriction of nitrogen or phosphorus in producing the polyester are carried out in a main culture in a polyester production medium. Incidentally, the microorganisms are generally cultured in a seed medium or a preculture medium to grow cells to a certain level prior to the main culture in the polyester production medium. In such a case, the same nutrient sources as described above may be used in the seed medium or the preculture medium. The culture temperature in these media may be the similar to the above polyester production medium, and the culture period is preferably 1 to 2 days, respectively.

Additionally, when the transformed microorganism is used, for example, antibiotics such as kanamycin, ampicillin and tetracycline, which correspond to a resistant gene exist in vectors, may be added to a preculture medium.

In the present invention, a method of collecting the copolyester from bacterial cells is not particularly restricted, and the conventional solvent extraction methods, physical cell disruption, and chemical treatment, etc., for example, the following methods may be used. After completion of a culture, cells are separated from a culture broth by using a centrifuge and the like, then the cells are washed with distilled water, methanol and the like, and dried. A polyester is extracted using organic solvents such as chloroform. Cell components are removed from the polyester-containing organic solvent by filtration, etc., and poor solvent such as methanol or hexane is added to the filtrate to precipitate the polyester. The supernatant is removed by filtration or centrifugation, and dried to collect a polyester.

The monomeric unit of the polyester obtained can be analyzed by gas chromatography or nuclear magnetic resonance spectrometry, for instance.

BRIEF DESCRIPTION OF THE DRAWINGS

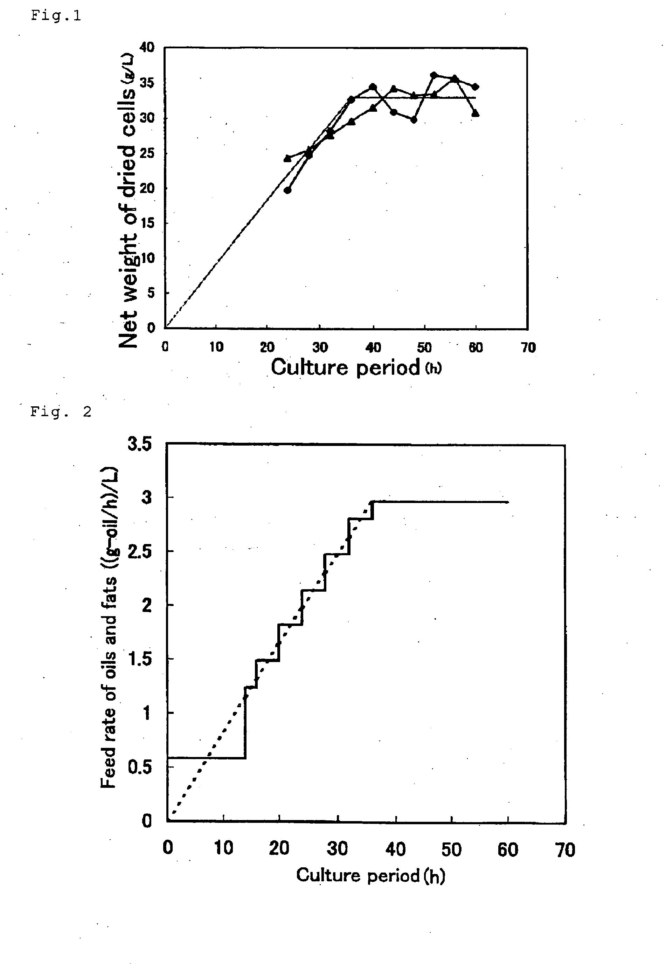

Fig. 1 shows changes in measured values (▲ and ♦ in the Fig.) of the net weight of dries cells (g/L) per unit culture broth in two preliminary experiments using palm kernel olein oil, and growth curves of the net weight of dries cells per unit culture broth obtained therefrom.

Fig. 2 shows theoretical values of a feed rate ((g-oil/h)L) per unit culture broth of palm kernel olein oil (broken line) when a specific substrate feed rate is set to be 0.09 (g; oil) $\times$ (g; net weight of dried cells)$^{-1}$ $\times$ (hour)$^{-1}$, and a pattern of changes of a feed rate per unit culture broth of an oil or fat actually fed in Examples(solid line).

BEST MODE FOR CARRYING OUT THE INVENTION

The present invention is further illustrated by the following examples. In the respective following Examples, P(3HB-co-3HH) was produced as a 3HH-containing copolyester. The technical field of the present invention, of course, is not limited to these Examples and is not limited to the production of P(3HB-co-3HH).

For example, it is possible to produce a 3HH-containing copolymer other than P(3HB-co-3HH) by changing the species of a microorganism or a polyester polymerase gene to be used, by using another oil or fat as a carbon source or by adding a specific fatty acid.

It is to be noted that the unit of the specific substrate feed rate is as follows. (g; oil or fat) $\times$ (g; net weight of dried cells)$^{-1}$ $\times$ (hour)$^{-1}$

(Example 1)

Ralstonia eutropha strain PHB-4/pJRDEE32d13 (T. Fukui., Y. Doi., Appl. Microbiol. Biotechnol., 49, 333-336 (1998)) (hereinafter, referred to briefly as "Red 13" strain) was cultured as follows. Incidentally, the Red 13 strain is deposited internationally to the National Institute of Advanced Industrial Science and Technology, International Patent Organism Depositary (Central 6, 1-1-1, Higashi, Tsukuba, Ibaraki, JAPAN) on the date of August 7, 1997 under the name of Alcaligenes eutrophus AC 32 in an accession number of FERM BP-6038 under Budapest treaty.

The composition of the seed medium was made to comprise 1 w/v% Meat-extract, 1 w/v% Bacto-trypton, 0.2 w/v% Yeast-extract, 0.9 w/v% $Na_2PO_4\cdot12H_2O$, 0.15 w/v% $KH_2PO_4$ and pH 6.8.

The composition of the preculture medium was made to comprise 1.1 w/v% $Na_2PO_4\cdot12H_2O$, 0.19 w/v% $KH_2PO_4$, 1.29 w/v% $(NH_4)_2SO_4$, 0.1 w/v% $MgSO_4\cdot7H_2O$, 2.5 w/v% palm W olein oil, 0.5 v/v% trace metal salt solution (1.6 w/v% $FeCl_3\cdot6H_2O$, 1 w/v% $CaCl_2\cdot2H_2O$, 0.02 w/v% $CoCl_2\cdot6H_2O$, 0.016 w/v% $CuSO_4\cdot5H_2O$ and 0.012 w/v% $NiCl_2\cdot6H_2O$ were dissolved in 0.1 N hydrochloric acid), and $5\times10^{-6}$ w/v% kanamycin.

The composition of the polyester production medium was made to comprise 0.385 w/v% $Na_2PO_9\cdot12H_2O$, 0.067 w/v% $KH_2PO_4$, 0.291 w/v% $(NH_4)_2SO_4$, 0.1 w/v% $MgSO_4\cdot7H_2O$, 0.5 v/v% trace metal salt solution (1.6 w/v% $FeCl_3\cdot6H_2O$, 1 w/v% $CaCl_2\cdot2H_2O$, 0.02 w/v% $CoCl_2\cdot6H_2O$, 0.016 w/v% $CuSO_4\cdot5H_2O$ and 0.012 w/v% $NiCl_2\cdot6H_2O$ dissolved in 0.1 N hydrochloric acid) and 0.05 w/v% BIOSPUREX200K (antifoaming agent: product of Cognis Japan Ltd.) . Palm kernel olein oil, which is a low melting point fraction fractionated from palm kernel oil, was used as a carbon source and fed at a specific substrate feed rate of 0.08, 0.09 and 0.10 (g; oil or fat) $\times$ (g; net weight of dried cells)$^{-1}$ $\times$ (hour)$^{-1}$, respectively, throughout the whole culture period.

The growth curve of the net weight of dried cells, which forms the basis for the control of the specific substrate feed rate, was prepared based on the results obtained from the culture conducted as a preliminary experiment, in which addition amount of palm kernel olein oil was adjusted in accordance with the observation of the thickness of the oil layer in the culture supernatant. More specifically, from the data on the changes in the net weight of dried cells per unit culture broth in the preliminary experiment, the one showing a linear growth until 36-hour culture and thereafter the net weight of dried cells per unit culture broth maintained at a constant value was employed, as shown in Fig. 1.

Fig. 2 and Table 1 show theoretical values of the feed rate per unit culture broth of palm kernel olein oil (broken line) and a pattern of stepwise changes in the feed rate per unit culture broth of the oil or fat actually fed in the following Examples (solid line) in the case the specific substrate feed rate is set to be 0.09 (g; oil or fat) $\times$ (g; net weight of dried cells)$^{-1}$ $\times$ (hour)$^{-1}$ using the growth curve of the net weight of dries cells obtained in Fig. 1.

Incidentally, in Fig. 1, 2 and the following Tables 1 to 8, L represents culture broth (total content of medium, cells and oil or fat) as liter, and h represents hour.

Table 1

| Culture period (hour) | Feed rate of oils and fats ((g-oil/h)/L) | Stepwise change of feed rate ((g-oil/h)/L) |
|---|---|---|
| 0 | 0.00 | 0.58 |
| 14 | 1.16 | 1.24 |
| 16 | 1.32 | 1.49 |
| 20 | 1.65 | 1.82 |
| 24 | 1.98 | 2.14 |
| 28 | 2.31 | 2.48 |
| 32 | 2.64 | 2.81 |
| 36 | 2.97 | 2.97 |
| 40 | 2.97 | 2.97 |
| 44 | 2.97 | 2.97 |
| 48 | 2.97 | 2.97 |
| 52 | 2.97 | 2.97 |
| 56 | 2.97 | 2.97 |
| 60 | 2.97 | 2.97 |

A glycerol stock (50 $\mu$l) of Red 13 strain was inoculated to a seed medium (10 ml) and cultured for 24 hours. Then, the resultant was inoculated in a ratio of 0.2 v/v% to a 5 L jar fermentor (MDL-500 type, product of B.E. Marubishi Co., Ltd.) containing 3 L of the preculture medium. The running condition was set to be a culturing temperature of 30 °C, a stirring rate of 500 rpm, an aeration rate of 1.8 L/min. The culture was carried out for 28 hours with controlling pH between 6.7 and 6.8. 7% ammonium hydroxide solution was used for the pH control.

The polyester production culture was carried out as the following. 10 L jar fermentor (MDL-1000 type, product of B.E. Marubishi Co., LTD.) containing 6 L of the production medium was inoculated with 1.0 v/v% of the preculture seed. The running condition was set to be a culturing temperature of 28 °C, a stirring rate of 400 rpm, an aeration rate of 3.6 L/min and pH was controlled of between 6. 7 and 6.8% ammonium hydroxide solution was used for the pH control. 14% ammonium hydroxide solution was used for the pH control. The culture was carried out for 60 hours, samplings were conducted at every 4 hours after 16 hours of the culture, cells were collected by a centrifugation, washed with methanol, lyophilized and the weight of the dried cells was measured.

To approximately 1 g of the dried cell obtained was added with 100 ml of chloroform, and the mixture was stirred overnight at a room temperature to extract a polyester within cells. After the cell residue was filtered off, the resultant was concentrated by an evaporator until the total content to be 30 ml, approximately 90 ml of hexane was added gradually with stirring slowly, and allowed to stand for 1 hour. The precipitated polyester was filtered off, and dried in vacuo for 3 hours at 50°C. The weight of the dried polyester was measured and the content of the polyester within the cells were calculated.

Approximately 20 mg of the obtained dried polyester was added with 2 ml of a sulfuric acid-methanol mixture (15: 85) and 2 ml of chloroform, and the container was sealed. The obtained mixture was heated at 100°C for 140 minutes to obtain methylester of a polyester decomposition product. After cooling, 1.5 g of sodium bicarbonate was added by little and little to neutralize, and the mixture was left until generation of carbon dioxide stops. After 4 ml of diisopropyl ether was added and well-mixed, the mixture was centrifuged to analyze a composition of a monomeric unit of the polyester decomposition product in a supernatant by a capillary gas chromatography. The used gas chromatograph was GC-17A produced by Shimadzu Corporation, and the used capillary column was NEUTRA BOND-1 produced by GL Science Co., LTD. (column length 25 m, column inner diameter 0.25 mm and liquid film thickness 0.4 $\mu$m). The temperature condition was set to be raising 8°C/min from 100°C of an initial temperature to 200°C, and further 30°C/min from 200°C to 290°C.

Table 2 shows the influence on the composition ratio of copolyester and the productivity by the control of the specific substrate feed rate.

Table 2

P(3HB-co-3HH) produced (g/L)

| Specific substrate feed rate | Culture 24-h | Culture 36-h | Culture 48-h | Culture 60-h |
| --- | --- | --- | --- | --- |
| 0.08 | 2 | 15 | 40 | 63 |
| 0.09 | 3 | 18 | 43 | 66 |
| 0.10 | 3 | 21 | 46 | 68 |

3HH content (mol%)

| Specific substrate feed rate | Culture 24-h | Culture 36-h | Culture 48-h | Culture 60-h |
| --- | --- | --- | --- | --- |
| 0.08 | 18.2 | 15.1 | 12.4 | 11.1 |
| 0.09 | 15.8 | 12.2 | 10.3 | 8.9 |
| 0.10 | 13.2 | 10.2 | 8.5 | 7.5 |

From the results, it was found that, although the productivity was slightly deteriorated as the specific substrate feed rate was controlled to be lower, the 3HH content of the copolyester was enhanced. In addition, it was observed that foam generation during the culture was suppressed more preferably as the specific substrate feed rate was set to be lower.

(Example 2)

A culture was carried out in the same medium and conditions as in Example 1 except that soybean oil was used instead of palm kernel olein oil. The results shown in Table 3 were obtained.

Table 3

P(3HB-co-3HH) produced (g/L)

| Specific substrate feed rate | Culture 24-h | Culture 36-h | Culture 48-h | Culture 60-h |
| --- | --- | --- | --- | --- |
| 0.08 | 2 | 33 | 55 | 62 |
| 0.09 | 4 | 39 | 60 | 64 |
| 0.10 | 3 | 41 | 60 | 68 |

3HH content (mol%)

| Specific substrate feed rate | Culture 24-h | Culture 36-h | Culture 48-h | Culture 60-h |
| --- | --- | --- | --- | --- |
| 0.08 | 13.2 | 6.7 | 5.3 | 4-1 |
| 0.09 | 12.9 | 5.8 | 4.2 | 3.4 |
| 0.10 | 13.8 | 4.9 | 3.3 | 3.2 |

As shown in Table 3, it was found that soybean oil, similarly to palm kernel olein oil, enhanced the 3HH content in polyester by controlling the specific substrate feed rate to be low. However, when the 3HH contents under the same conditions of the specific substrate feed rate were compared between soybean oil and palm kernel olein oil used in Example 1, the content was markedly lower in the case of soybean oil. Therefore, it was found that a difference in the 3HH content was caused by a difference of an oil or fat used as a substrate even when the specific substrate feed rate was controlled at the same value.

Foaming was preferably suppressed when the specific substrate feed rate was set to the lowest value of 0.08 (g; oil or fat) $\times$ (g; net weight of dried cells)$^{-1}$ $\times$ (hour)$^{-1}$ as in Example 1.

(Example 3)

A culture was carried out in the same medium and conditions as in Example 1 except that corn oil, cotton seed oil, palm kernel oil, coconut oil, peanut oil and palm W olein oil were used instead of palm kernel olein oil and that the specific substrate feed rate were controlled at 0.06 (g; oil or fat) $\times$ (g; net weight of dried cells)$^{-1}$ $\times$ (hour)$^{-1}$ and 0.12 (g; oil or fat) $\times$ (g; net weight of dried cells)$^{-1}$ $\times$ (hour)$^{-1}$ for the respective oils. The results shown in Table 4 were obtained. Table 4 summarizes the influence of the specific substrate feed rate on the productivity of copolyester and the 3HH content for the respective oils (the results obtained at 60-hour culture were compared).

Table 4

| Oils and fats | Specific substrate feed rate 0.06 | | Specific substrate feed rate 0.12 | |
|---|---|---|---|---|
| | P(3HB-co-3HH) produced (g/L) | 3HH content (mol%) | P(3HB-co-3HH) produced (g/L) | 3HH content (mol%) |
| Corn oil | 63 (g/L) | 3.2 | 68 | 2.7 |
| Cottonseed oil | 48 | 3.3 | 52 | 2.7 |
| Palm W olein oil | 60 | 3.3 | 62 | 3 |
| Palm kernel oil | 45 | 7 | 48 | 6.8 |
| Coconut oil | 41 | 13.8 | 43 | 13.5 |
| Peanut oil | 43 | 5.2 | 45 | 4.6 |

As shown in Table 4, for all oils and fats, it was found that the 3HH content in copolyester was enhanced by controlling the specific substrate feed rate to be low, although the productivity of polyester was slightly deteriorated. Regarding the 3HH compositions achieved at 60-hour culture, there were apparent differences depending on oils and fats used as a substrate, even when the specific substrate feed rate was controlled at the same value. It was found that P(3HB-co-3HH) with a relatively high 3HH content of 7 to 14 mol% was obtained from the lauric oils and fats, i.e. coconut oil and palm kernel oil, and P(3HB-co-3HH) with a relatively low 3HH content of 3 to 6 mol% was obtained from corn oil, cottonseed oil, palm W olein oil and peanut oil. The results shown in Table 4 suggest that it is possible to obtain a copolyester having the desired 3HH content while securing the high productivity by selecting the species or a set value of the specific substrate feed rate and appropriately combining these two parameters.

(Example 4)

A culture was carried out in the same medium and conditions as in Example 1 except that three mixed oils and fats of palm kernel olein oil and soybean oil, that is, mixed oil A (palm kernel olein oil/ soybean oil = 75/ 25 (v/v)), mixed oil B (palm kernel olein oil/ soybean oil = 50/ 50 (v/v)), and mixed oil C (palm kernel olein oil/ soybean oil = 25/ 75 (v/v) were used as substrates. The results shown in Tables 5 to 7 were obtained. Tables 5 to 7 summarize the influence of the specific substrate feed rate on the productivity of polyester and the 3HH content for the respective mixed oils and fats.

Table 5
Mixed oil A(Palm kernel olein oil/soy bean oil =75/25(v/v)) P(3HB-co-3HH) produced (g/L)

| Specific substrate feed rate | Culture 24-h | Culture 36-h | Culture 48-h | Culture 60-h |
|---|---|---|---|---|
| 0.08 | 3 | 16 | 39 | 64 |
| 0.09 | 2 | 20 | 44 | 66 |
| 0.10 | 3 | 23 | 48 | 69 |

(continued)

| 3HH content (mol%) | | | | |
| --- | --- | --- | --- | --- |
| Specific substrate feed rate | Culture 24-h | Culture 36-h | Culture 48-h | Culture 60-h |
| 0.08 | 16.9 | 13.8 | 11.1 | 10.3 |
| 0.09 | 14.1 | 10.8 | 8.6 | 7.5 |
| 0.10 | 11 | 8.6 | 7.5 | 7 |

Table 6

Mixed oil B(Palm kernel olein oil/soy bean oil =50/50(v/v)) P(3HB-co-3HH) produced (g/L)

| Specific substrate feed rate | Culture 24-h | Culture 36-h | Culture 48-h | Culture 60-h |
| --- | --- | --- | --- | --- |
| 0.08 | 2 | 15 | 37 | 65 |
| 0.09 | 3 | 21 | 42 | 66 |
| 0.10 | 2 | 22 | 48 | 70 |
| 3HH content (mol%) | | | | |
| Specific substrate feed rate | Culture 24-h | Culture 36-h | Culture 48-h | Culture 60-h |
| 0.08 | 15.9 | 12.6 | 10.2 | 8.1 |
| 0.09 | 13.6 | 9.8 | 7.9 | 6.8 |
| 0.10 | 9.9 | 7.4 | 6.8 | 6.3 |

Table 7

Mixed oil C(Palm kernel olein oil/soy bean oil =25/75(v/v)) P(3HB-co-3HH) produced (g/L)

| Specific substrate feed rate | Culture 24-h | Culture 36-h | Culture 48-h | Cultureh 60-h |
| --- | --- | --- | --- | --- |
| 0.08 | 3 | 12 | 36 | 66 |
| 0.09 | 3 | 23 | 43 | 66 |
| 0.10 | 4 | 23 | 47 | 68 |
| 3HH content (mol%) | | | | |
| Specific substrate feed rate | Culture 24-h | Culture 36-h | Culture 48-h | Culture-h 60-h |
| 0.08 | 12.1 | 9.2 | 7.2 | 6.3 |
| 0.09 | 11.6 | 8.3 | 6.2 | 5.7 |
| 0.10 | 8.7 | 6.8 | 5.8 | 5.2 |

As shown in Tables 5 to 7, it was found that the 3HH content of polyester was enhanced in all the mixed oils and fats by controlling the specific substrate feed rate to be low although the productivity of polyester was slightly deteriorated. As for the 3HH content, it was found that the 3HH content was enhanced with increase of the ratio of palm kernel olein oil in mixed oils and fats. This can also be said from the results of Tables 5 to 7 and the cases where palm kernel olein oil and soybean oil were used in 100% (Table 2 in Example 1 and Table 3 in Example 2). The results shown in Tables 5 to 7 suggest that it becomes possible to obtain a polyester having the desired 3HH content while securing the high productivity by combining the species of oils or fats and the control value of the specific substrate feed rate described in Example 3, and in addition to that, by mixing a plurality of oils and fats and further by adjusting the mixing ratio.

(Example 5)

A culture was carried out in the same medium and conditions as in Example 1 except that palm kernel olein oil was used as a carbon source and the specific substrate feed rate was controlled at 0.09 (g; oil or fat) $\times$ (g; net weight of dried cells)$^{-1}$ $\times$ (hour)$^{-1}$ during the cell growth phase, which is until 36-hour culture, and thereafter at 0.08 (g; oil) $\times$ (g; net weight of dried cells)$^{-1}$ $\times$ (hour)$^{-1}$ during the polyester accumulation phase.

| Table 8 | | | | |
|---|---|---|---|---|
| | Culture 24-h | Culture 36-h | Culture 48-h | Culture 60-h |
| P(3HB-co-3HH) production amount (g/L) | 3 | 17 | 42 | 65 |
| 3HH content (mol%) | 16.0 | 13.0 | 12.5 | 12.3 |

It is obvious when the results shown in Table 8 are compared with those of Example 1 shown in Table 2 that by culturing with a slightly reduced specific substrate feed rate during the polyester accumulation phase, the high 3HH content which is equivalent to or higher than that obtained in the culture with the specific substrate feed rate being controlled at 0.08 (g; oil or fat) $\times$ (g; net weight of dried cells)$^{-1}$ $\times$ (hour)$^{-1}$ throughout the whole culture period was attained without decreasing the productivity as compared with the culture with the specific substrate feed rate being controlled at 0.09 (g; oil or fat) $\times$ (g; net weight of dried cells)$^{-1}$ $\times$ (hour)$^{-1}$ throughout the whole culture period.

INDUSTRIAL APPLICABILITY

In the method according to the present invention, it becomes possible to control optionally the composition of the 3HH-containing copolyester, a biodegradable polymer, which makes drastic changes in the properties of the copolyester, as well as to stably obtain a high productivity by selecting a control value for the specific substrate feed rate of an oil or fat used as a carbon source, and further by making a combination of the control value for the specific substrate feed rate with the species of the oils or fats used. Therefore, a 3HH-containing copolyester having a wide range of applications may be produced and provided industrially in low cost. In addition, foaming attributed to an excessive supply of oils and fats may be preferably suppressed by controlling the specific substrate feed rate, thereby a culture may be stabilized.

**Claims**

1. A culture method in producing a copolyester by a microorganism
   which comprises controlling a specific substrate feed rate of an oil or fat to be used as a carbon source at a constant value throughout the whole culture period,
   wherein the microorganism is selected from the group consisting of microorganisms belonging to the genus Ralstonia, the genus Pseudomonas, the genus Aeromonas, the genus Alcaligenes and the genus Escherichia,
   wherein the copolyester contains 3-hydroxyhexanoic acid unit, and
   wherein the specific substrate feed rate is calculated according to the following formula:

```
    (Specific substrate feed rate) = (Supply amount of an oil
or fat per hour (g/h)) / [(Whole cell weight (g)) - (Polyester
contained in cells (g))].
```

2. A culture method in producing a copolyester by a microorganism
   which comprises applying a different specific substrate feed rate of an oil or fat used as a carbon source between a cell growth phase and a polyester accumulation phase in a culture and controlling the rate at a constant value during the respective phases,
   wherein the microorganism is selected from the group consisting of microorganisms belonging to the genus Ralstonia, the genus Pseudomonas, the genus Aeromonas, the genus Alcaligenes and the genus Escherichia,
   wherein the copolyester contains 3-hydroxyhexanoic acid unit, and
   wherein the specific substrate feed rate is calculated according to the following formula:

```
    (Specific substrate feed rate) = (Supply amount of an oil
or fat per hour (g/h)) / [(Whole cell weight (g)) - (Polyester
contained in cells (g))].
```

3. The culture method according to Claim 1 or 2
which comprises controlling the composition of the produced copolyester by selecting the species and/or the control
value for the specific substrate feed rate.

4. The culture method according to any one of Claims 1 to 3,
wherein the oil or fat used as a carbon source contains at least one oil or fat selected from the group consisting of
soybean oil, corn oil, cottonseed oil, palm oil, palm kernel oil, coconut oil and peanut oil, and fractionated oils obtained
by fractionating these oils.

5. The culture method according to any one of Claims 1 to 4,
wherein the oil or fat used as a carbon source contains lauric acid in the constituent fatty acids, and
the culture is carried out under the condition phosphorus being restricted.

6. The culture method according to any one of Claims 1 to 5,
wherein the microorganism is a transformed microorganism into which a polyester polymerase gene is incorporated.

**Patentansprüche**

1. Kultivierungsverfahren in der Herstellung eines Copolyesters durch einen Mikroorganismus,
das die Steuerung einer spezifischen Substratzufuhrrate eines Öles oder Fettes, das als Kohlenstoffquelle eingesetzt
wird, mit einem konstanten Wert über die gesamte Kultivierungsdauer umfasst,
worin der Mikroorganismus aus der Gruppe ausgewählt ist, die aus Mikroorganismen besteht, die zur Gattung
Ralstonia, zur Gattung Pseudomonas, zur Gattung Aeromonas, zur Gattung Alcaligenes und zur Gattung Escherichia
gehören,
worin der Copolyester eine 3-Hydroxyhexansäureeinheit enthält, und
worin die spezifische Substratzufuhrrate gemäß der folgenden Formel berechnet wird:

```
    (Spezifische Substratzufuhrrate) = (bereitgestellte
    Menge eines Öls oder Fettes pro Stunde (g/Std.) /
    [(Gesamt-Zellengewicht (g)) - (in den Zellen enthaltener
    Polyester (g))].
```

2. Kultivierungsverfahren in der Herstellung eines Copolyesters durch einen Mikroorganismus,
das die Anwendung einer unterschiedlichen spezifischen Substratzufuhrrate eines Öls oder Fettes, das als Kohlenstoffquelle eingesetzt wird, zwischen einer Zellwachstumsphase und einer Polyesterakkumulierungsphase in
einer Kultur, sowie das Regeln der Rate auf einen konstanten Wert während der jeweiligen Phase umfasst,
worin der Mikroorganismus aus der Gruppe ausgewählt ist, die aus Mikroorganismen besteht, die zur Gattung
Ralstonia, zur Gattung Pseudomonas, zur Gattung Aeromonas, zur Gattung Alcaligenes und zur Gattung Escherichia
gehören,
worin der Copolyester eine 3-Hydroxyhexansäureeinheit enthält, und
worin die spezifische Substratzufuhrrate gemäß der folgenden Formel berechnet wird:

```
(Spezifische Substratzufuhrrate) = (bereitgestellte
Menge eines Öls oder Fettes pro Stunde (g/Std.) /
[(Gesamt-Zellengewicht (g)) - (in den Zellen enthaltener
Polyester (g))].
```

3. Kultivierungsverfahren gemäß Anspruch 1 oder 2, das die Steuerung der Zusammensetzung des hergestellten Copolyesters durch Auswahl der Spezies und/oder des geregelten Werts für die spezielle Substratzufuhrrate umfasst.

4. Kultivierungsverfahren gemäß irgendeinem der Ansprüche 1 bis 3, worin das als Kohlenstoffquelle eingesetzte Öl oder Fett mindestens ein Öl oder Fett ausgewählt aus der Gruppe bestehend aus Sojabohnenöl, Maisöl, Baumwollsamenöl, Palmöl, Palmkernöl, Kokosnussöl, Erdnussöl und fraktionierten Ölen, die durch Fraktionieren dieser Öle erhalten wurden, umfasst.

5. Kultivierungsverfahren gemäß irgendeinem der Ansprüche 1 bis 4,
worin das als Kohlenstoffquelle eingesetzte Öl oder Fett in den aufbauenden Fettsäuren Laurylsäure enthält, und die Kultivierung unter der Bedingung durchgeführt wird, dass Phosphor eingeschränkt ist.

6. Kultivierungsverfahren gemäß irgendeinem der Ansprüche 1 bis 5, worin der Mikroorganismus ein transformierter Mikroorganismus ist, in den ein Polyesterpolymerase-Gen eingebracht ist.

**Revendications**

1. Procédé de culture dans la production d'un copolyester par un microorganisme
qui comprend le fait de réguler un débit d'alimentation en substrat spécifique d'une huile ou d'une matière grasse à utiliser en tant que source de carbone à une valeur constante pendant toute la période de culture,
dans lequel le microorganisme est choisi dans le groupe constitué de microorganismes appartenant au genre *Ralstonia,* au genre *Pseudomonas,* au genre *Aeromonas,* au genre *Alcaligenes* et au genre *Escherichia,*
dans lequel le copolyester contient une unité d'acide 3-hydroxyhexanoïque, et
dans lequel le débit d'alimentation en substrat spécifique est calculé selon la formule suivante :

```
(Débit d'alimentation en substrat spécifique) =
(quantité d'alimentation d'une huile ou d'une matière
grasse par heure (g/h))/[(poids de cellules entières (g)) -
(Polyester contenu dans les cellules (g))].
```

2. Procédé de culture dans la production d'un copolyester par un microorganisme
qui comprend le fait d'appliquer un débit d'alimentation en substrat spécifique différent d'une huile ou d'une matière grasse utilisée en tant que source de carbone entre une phase de croissance cellulaire et une phase d'accumulation de polyester dans une culture et de réguler le débit à une valeur constante pendant les phases respectives,
dans lequel le microorganisme est choisi dans le groupe constitué de microorganismes appartenant au genre *Ralstonia,* au genre *Pseudomonas,* au genre *Aeromonas,* au genre *Alcaligenes* et au genre *Escherichia,*
dans lequel le copolyester contient une unité d'acide 3-hydroxyhexanoïque, et
dans lequel le débit d'alimentation en substrat spécifique est calculé selon la formule suivante :

(Débit d'alimentation en substrat spécifique) = (Quantité d'alimentation d'une huile ou d'une matière grasse par heure (g/h))/((poids de cellules entières (g)) - (Polyester contenu dans les cellules (g))].

3. Procédé de culture selon la revendication 1 ou 2, qui comprend le fait de réguler la composition du copolyester produit en sélectionnant les espèces et/ou la valeur de régulation pour le débit d'alimentation en substrat spécifique.

4. Procédé de culture selon l'une quelconque des revendications 1 à 3, dans lequel l'huile ou la matière grasse utilisée en tant que source de carbone contient au moins une huile ou une matière grasse choisie dans le groupe constitué de l'huile de soja, de l'huile de maïs, de l'huile de graines de coton, de l'huile de palme, de l'huile de palmiste, de l'huile de noix de coco et de l'huile d'arachide et d'huiles fractionnées obtenues par fractionnement de ces huiles.

5. Procédé de culture selon l'une quelconque des revendications 1 à 4, dans lequel l'huile ou la matière grasse utilisée en tant que source de carbone contient de l'acide laurique dans les acides gras constitutifs, et la culture est effectuée à condition que le phosphore soit limité.

6. Procédé de culture selon l'une quelconque des revendications 1 à 5, dans lequel le microorganisme est un microorganisme transformé dans lequel un gène de polyester-polymérase est incorporé.

Fig.1

Fig. 2

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP SHO57150393 B
- JP SHO59220192 B
- JP HEI11500008 B
- JP SHO63269989 B
- JP HEI779705 B

- JP HEI0593049 B
- JP HEI07265065 B
- JP HEI10108682 B
- JP 2001340078 A

### Non-patent literature cited in the description

- **POIRIER, Y. ; NAWRATH C. ; SOMERVILLE C.** *BIO/TECHNOLOGY,* 1995, vol. 13, 142-150
- **Y. DOI ; S. KITAMURA ; H. ABE.** *Macromolecules,* 1995, vol. 28, 4822-4823
- **T. FUKUI ; Y. DOI.** *J. Bacteriol.,* 1999, vol. 179 (15), 4821-4830
- **T. FUKUI ; Y. DOI.** *Appl. Microbiol. Biotechnol.,* 1998, vol. 49 (3), 333-336

- **S.H. LEE ; D.H. OH ; W.S. AHN.** *Biotechnol. Bioeng.,* 2000, vol. 67 (3), 240-244
- **I.K.P. TAN ; K.S. KUMAR ; M. THEANMALAR ; S.N. GAN ; B. GORDON III.** *Appl. Microbiol. Biotechnol.,* 1997, vol. 47, 207-211
- **R.D. ASHBY ; T.A. FOGLIA.** *Appl. Microbiol. Biotechnol.,* 1998, vol. 49, 431-437
- **T. FUKUI. ; Y. DOI.** *Appl. Microbiol. Biotechnol.,* 1998, vol. 49, 333-336